# EUROPEAN PATENT APPLICATION

(11) **EP 4 574 189 A1**
(43) Date of publication of application: **25.06.2025**
(21) Application number: 23307366.7
(22) Date of filing: 22.12.2023
(51) Int. Cl.: A61M 5/315, A61M 5/32

(54) **SAFETY SYSTEM FOR A SYRINGE AND DRUG DELIVERY DEVICE INCLUDING SUCH A SAFETY SYSTEM**

(71) Applicant: NEMERA LA VERPILLIERE, 38290 La Verpillière (FR)
(72) Inventor: BILLON, Maxime, 10110 Polisy (FR); DING, David, 38110 Saint-Didier-de-la-Tour (FR); MEGARD, Thomas, 69230 Saint-Genis-Laval (FR)
(74) Representative: Lavoix

(57) **Abstract**

A safety system (40) for a syringe (4) has a longitudinal axis (A40) and comprises a body (34), provided with at least one first coupling member (80) arranged on an outer surface (S34) of the body, and a plunger rod cover (36), provided with at least a second coupling member (72). The body (34) and the plunger rod cover (36) cannot move axially with respect to each other, when the coupling members (80, 72) are in a coupled configuration. The body (34) and the plunger rod cover (36) are movable with respect to each other at least in the axial direction, when the coupling members are in a decoupled configuration. The coupling members (80, 72) decouple via a relative rotation (R_{D}) around the longitudinal axis (A40). At least one coupling member (80) is configured to deform during a disconnection process where the coupling members decouple via the relative rotation (R_{D}).

## Description

### TECHNICAL FIELD OF THE INVENTION

The present invention concerns a safety system for a syringe, in particular a safety system of a drug delivery device. The present invention also concerns a drug delivery device, including, amongst others, such a safety system.

Whatever the embodiment envisaged, the drug delivery device according to the invention can, for example, be used to administer a drug in liquid form to a patient in order to treat a wide variety of pathologies such as, for example, autoimmune diseases, or chronic diseases such as diabetes or associated disorders, cancers, hormonal insufficiency, macular degeneration, inflammation, atherosclerosis, rheumatoid arthritis, coronary syndromes, thromboembolic diseases, etc.

Thus, the term "drug" designates any liquid formulation capable of being administered in the sytinge. The formulation can be, for example, a solution, a gel or a fine suspension containing one or more active ingredients. These active ingredients may be, in particular, peptides (for example, insulin or GLP-1 , their derivatives or analogues, amylin or its derivatives or analogues, gastric inhibitory peptides or their analogues), proteins, glycoproteins or hormones (for example hormones from pituitary gland or hypothalamus, such as gonadotropin or gonadotropin releasing hormone, desmopressin, gonadorelin, triptorelin, terlipressin, leuprorelin, buserelin, goserelin, nafarelin, lutropin, menotropin, follicle -stimulating hormone and its analogues, follitropin, parathyroid hormone, teriparatide, abaloparatide or other analogues of parathyroid hormone, calcitonin, growth hormone, somatropin, etc., active ingredients derived from hormones or nucleotides (e.g. DNA, RNA, oligonucleotides), enzymes, polysaccharides (e.g. glycosaminoglycans, hyaluronic acids, heparins, low molecular weight heparins and their derivatives, or sulfated or forms poly-sulfated of these polysaccharides), vaccines, antibodies and their analogues (for example denosumab or panitumumab) or nutritional substances. The formulations may contain these active ingredients in the form of any pharmaceutically acceptable salt or solvate as well as any necessary and appropriate excipients.

When a syringe is inserted in a drug delivery device, a plunger rod is connected to a plunger sliding within a barrel of the syringe. The plunger rod extends in an axial direction, on a side of the barrel opposite to the needle of the syringe. There is therefore a risk of unintended displacement or distortion of the plunger rod and of the plunger before use of the syringe. This may occur during packing of the drug delivery device, during transportation and any other moment before use of the device. Such an unintended displacement of the plunger rod may even result in expelling a portion of a drug out of the syringe barrel. It may also actuate some peripherals of a drug delivery device, such as a tamper indicator or an electronic system or activate the safety system itself.

### BACKGROUND OF THE INVENTION

EP2047879A1 discloses a safety system for a syringe, which allows hiding the needle after injection. The syringe is held by a support element, axially movable with respect to a protective sheath. An initially compressed spring, which is released by opening a connection between the support element and the sheath, drives an axial movement of the syringe and the needle.

WO2011/135269A1 discloses a safety device, which includes an extender member for enlarging a gripping surface where a user of a drug delivery device may put his/her finger tips for easier injection.

WO2019/158547A1 discloses a medical injection device, which includes a syringe and a plunger protective shield removably mounted on a support member provided with two lateral arms. The plunger protective shield is associated with a needle protective shield and both elements have to be removed in order to carry out an injection. The plunger protective shield and the support member are preferably rigid, which may hinder their separation when the drug delivery device has to be used.

Thus, there is a need to provide a plunger rod cover for protecting a plunger rod before use of a syringe, this plunger rod cover being easily removed by a user, preferably via a simple movement, while providing an effective protection for the plunger rod when the plunger rod cover is mounted before use of the safety system with the syringe.

### SUMMARY OF THE INVENTION

The present invention aims at addressing these issues with a new safety system for a syringe, which allows efficiently protecting a plunger rod of the syringe and easily removing a plunger rod cover when the syringe needs to be used.

To this end, according to a first aspect, the invention concerns a safety system for a syringe, the safety system having a longitudinal axis and comprising
- a body provided with at least one first coupling member arranged on an outer surface of the body;
- a plunger rod cover provided with at least a second coupling member;
wherein
- the body and the plunger rod cover cannot move axially with respect to each other, when the first and second coupling members are in a coupled configuration;
- the body and the plunger rod cover are movable with respect to each other at least in the axial direction, when the first and second coupling members are in a decoupled configuration;
- the first and second coupling members decouple via a relative rotation around the longitudinal axis,
characterized in that at least one of the first and second coupling members is configured to deform during a disconnection process where the first and second coupling members decouple via the relative rotation.

Owing to the invention, at least one coupling member may deform, when these two coupling members decouple, during a disconnection process, so that this disconnection process can be easily implemented by a user of the syringe, in particular a patient whose physical condition does not allow him or her to exert an intense torque when rotating the plunger rod cover with respect to the body, in particular elderly people and chronic patients who perform self-injections. This may also apply to a healthcare professional who may have to repeat a disconnection process many times during a working shift. Thus, the invention provides easier manipulation of the plunger rod cover for disconnecting this cover from the body.

According advantageous aspects of the invention, the safety device might incorporate one or several of the following features, taken in any technically admissible combination:
- at least one of the coupling members is configured to deform elastically during a connection process, where the first and second coupling members couple via a relative rotation around the longitudinal axis.
- one of the first and second coupling members includes a radial protrusion, extending in a radial direction with respect to the longitudinal axis; and the other one of the coupling members includes a recess configured for accommodating the radial protrusion, when the body and the plunger rod cover are in their coupled configuration.
- the protrusion is provided on an elastically deformable tab.
- the two ends of the deformable tab are attached to a main portion of the body or of the plunger rod cover; the other one of the of the coupling members includes an axially extending structure equipped with a sliding surface configured to slide, during the disconnection process on a corresponding sliding surface of the deformable tab; and the recess is provided in the axially extending structure.
- the protrusion is provided at a free end of a U shaped deformable tab configured to be snapped into an empty space formed by the other one of the coupling member.
- the material of the part which carries the deformable tab is composed mainly of a copolymer of methyl methacrylate acrylonitrile butadiene and styrene (MABS), a copolymer of methyl methacrylate butadiene styrene (MMBS), polypropylene (PP), copolymer of acrylonitrile-butadiene-styrene (ABS), polyoxymethylene (POM), polyamide (PA), polybuthylene terephtalate (PBT), a polycarbonate (PC) or their mixtures.
- the plunger rod cover has the shape of a portion of a cylinder, opened on one angular sector.
- the plunger rod cover has a non-circular cross section.
- during the disconnection process, an angular amplitude of the rotation of the plunger rod cover with respect to the body is less than or equal to 100°, preferably between 30° and 50°.
- the safety system further comprises a sleeve and a spring, the spring being pre-compressed before activation of the safety system.
- the body comprises integral finger flanges or extended finger flanges attached to a gripping surface.
- the first coupling member belongs to the integral finger flanges or extended finger flanges.

According to a second aspect, the present invention concerns a drug delivery device comprising:
- a syringe with a syringe barrel; a plunger sliding within the barrel and a plunger rod configured to push the plunger during a dose delivery; and
- a safety system as mentioned here above.

Advantageously, the plunger rod comprises a head and a maximum transverse dimension of the head is smaller than an internal diameter of the plunger rod cover.

### BRIEF DESCRIPTION OF THE DRAWINGS

The invention will be better understood, based on the following description, which is given in correspondence with the appended figures and as an illustrative example, without restricting the object of the invention. In the annexed figures:
- [Fig.1] Figure 1 is a perspective view of a drug delivery device according to the invention, which includes a safety system according to the invention;
- [Fig.2] Figure 2 is an exploded perspective view of the drug delivery device of figure 1;
- [Fig.3] Figure 3 is a partial axial cut view taken along plane III in figure 1;
- [Fig.4] Figure 4 is a partial perspective view of a body of the drug delivery device of figures 1 to 3;
- [Fig.5] Figure 5 is a partial perspective view of a plunger rod cover of the drug delivery device of figures 1 to 3;
- [Fig.6] Figure 6 is a partial cut view, in the same plane as figure 3, when the drug delivery device is in a different configuration of use;
- [Fig.7] Figure 7 is a perspective view, similar to figure 1, for a drug delivery device according to a second embodiment of the invention, comprising a safety system according to a second embodiment of the invention;
- [Fig.8] Figure 8 is a perspective view of a body of the drug delivery device of figure 7; and
- [Fig.9] Figure 9 is a partial perspective view of a plunger rod cover of the drug delivery device of figure 7.

### DETAILED DESCRIPTION OF SOME EMBODIMENTS

In the foregoing description, a distal direction means a direction toward a needle or dispensing end of a syringe of a drug delivery device and a proximal direction means the opposite direction, i.e. the direction away from dispensing end or needle.

In the foregoing description, unless otherwise specified, the terms "axial", "axially", "radial" and "radially" are defined with respect to a longitudinal axis of a part, system or device. A direction or a dimension is axial or extends axially when it is parallel to such a longitudinal axis. A direction or a dimension is radial or extends radially when it is perpendicular to and crosses this longitudinal axis.

In the foregoing description, "elastic", "elastically" or "flexible" means that a part is capable of deforming reversibly under an effort, in standard conditions of use of a drug delivery device or syringe, in particular in a temperature range between 2°C and 42°C.

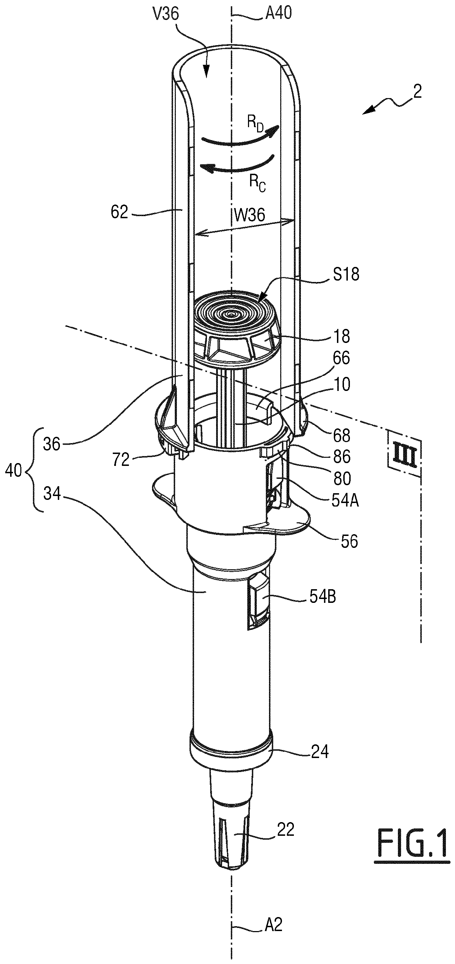

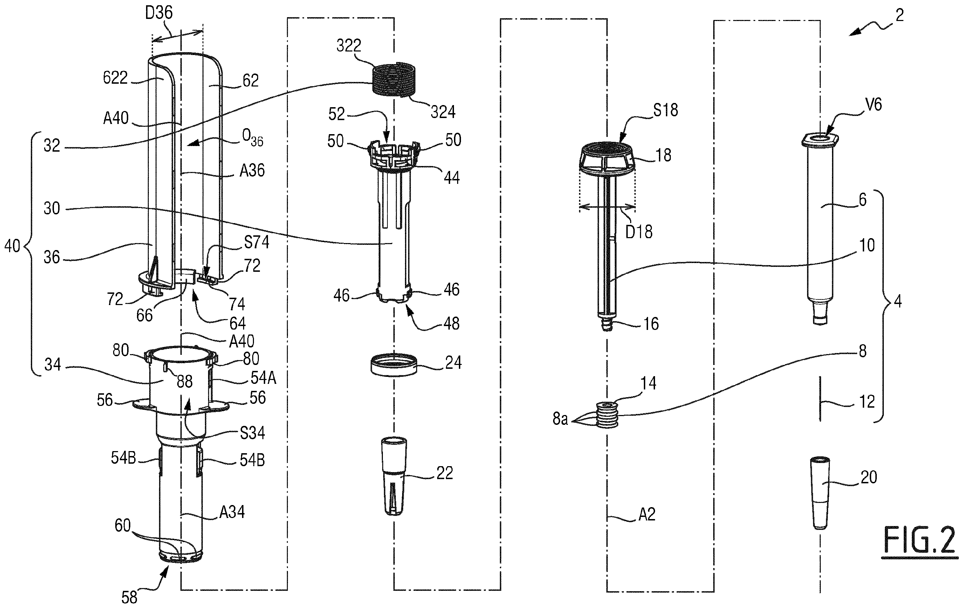

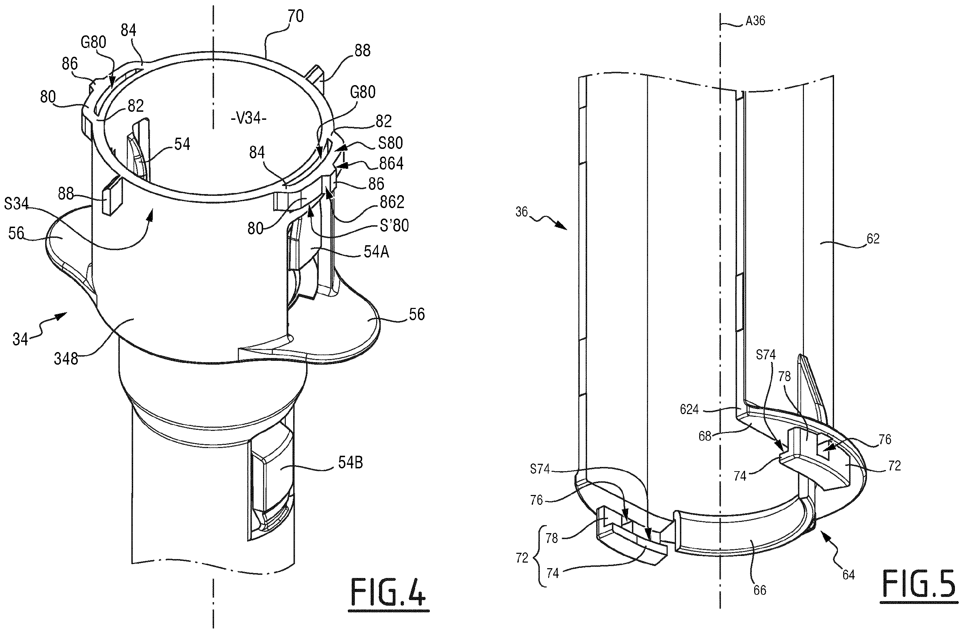

The drug delivery device 2 represented in figures 1 to 6 extends along a longitudinal axis A2 and includes a syringe 4, with a syringe barrel 6, a plunger 8, a plunger rod 10 and a needle 12.

The syringe barrel 6 is preferably made of glass or a plastic material and defines an internal volume V6 for receiving a drug in liquid form. The volume of the syringe barrek may be 0.5 ml (milliliter), 0.75 ml, 1 ml, 1.5 ml, 2.25 ml, 2.5 ml or any other volume suitable for the intended use of the drug delivery device.

The plunger 8 is preferably made of rubber and has some external ribs 8a for sliding within the internal volume V6 of the syringe barrel 6, in tight contact with an inner surface of this barrel.

In the embodiment of the figures, the plunger 8 has a central threaded opening 14 configured to be connected with a threaded distal end portion 16 of the plunger rod 10.

In a variant of the invention, the opening is not threaded before connection with the end portion 16.

Opposite its distal end portion 16, the plunger rod 10 has a proximal head 18. The proximal head 18 has a circular cross section. D18 denotes a maximum diameter of the head 18. This diameter is the maximum transverse dimension of the head 18 in a direction radial to the longitudinal axis A2.

In a variant non-represented in the drawings, the head 18 does not have a circular cross section. A maximum transverse dimension of this head can nevertheless be defined as the largest dimension of the head 18 in a direction radial to the longitudinal axis A2.

In the example of the figures, the needle 12 is fixedly mounted on the syringe barrel 6.

In a variant non-represented in the drawings, the needle 12 is attached to the syringe barrel 6 by releasable means, such as a Luer-lock connection.

S18 denotes a proximal end surface of the head 18 opposite to the end portion 16. This end surface S18 is configured to receive a pressure force exerted by the user, in order to push the plunger 8 toward the needle 12 and eject a liquid out of the syringe barrel 6, via the needle 12, when appropriate. The surface S18 may comprise a tactile structure with a distinctive shape which can be used for providing, for example, drug identification. Such a structure provides also better contact between the thumb and the surface S18 than a smooth surface.

The drug delivery device 2 also includes a needle cap 20, a needle cap remover 22 and a guiding member 24.

The drug delivery device 2 also includes a sleeve 30, a compression spring 32, a cylindrical body 34 and a plunger rod cover 36.

In an assembled configuration of the drug delivery device 2, all parts 4 to 36 are centered on the longitudinal axis A2.

The sleeve 30, the compression spring 32, the body 34 and the plunger rod cover 36 together belong to a safety system 40 for the syringe 4. A40 denotes a longitudinal axis of this safety system 40.

In assembled configuration of the drug delivery device 2, the two longitudinal axes A2 and A40 coincide.

In assembled configuration of the drug delivery device 2, the sleeve 30 is mounted around the syringe barrel 6, the body 34 is mounted around the sleeve 30 and the spring 32 is interposed, axially, between a portion of the sleeve 30 and a portion of the body 34, as explained here below.

The sleeve 30 has a flange 44.

322 denotes a proximal coil of the spring 32, that is the most proximal coil of the spring 32. 324 denotes the most distal coil of the spring 32, which is opposite to the most proximal coil 322.

The body 34 defines a cavity C34 for accommodating the spring 32. Radially to the longitudinal axis A40, this cavity extends between an inner wall 342 and an outer wall 344 of the body 34. These two walls are joined together at the level of a distal bottom 346 of the cavity C34. The walls 342 and 344 and the bottom 346 are formed by a main portion 348 of the body 34.

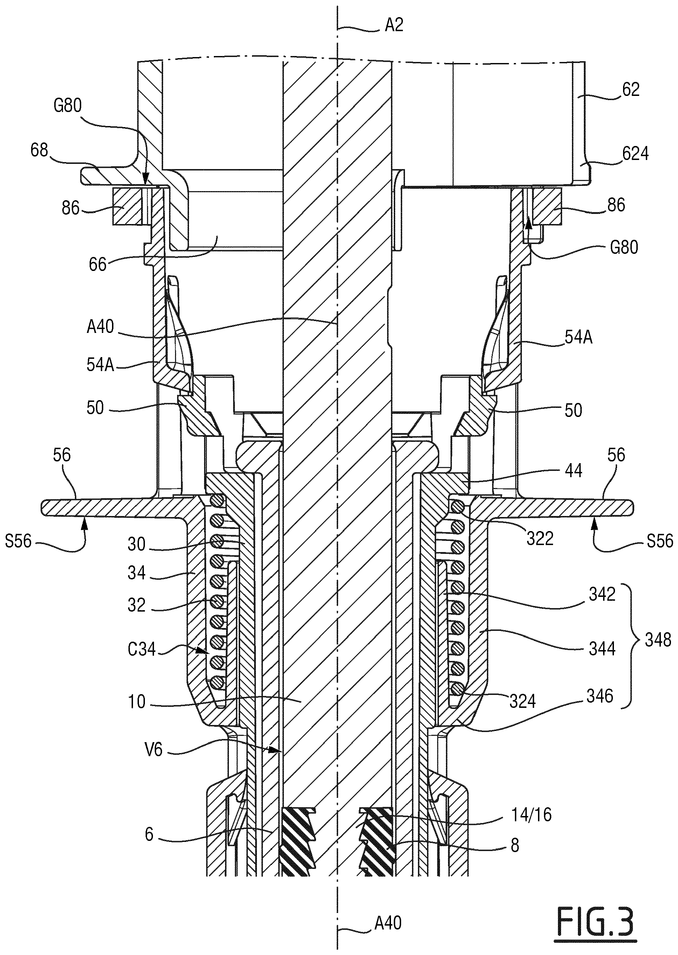

In the assembled configuration of the drug delivery device 2 represented in figures 1 and 3, the spring 32 is compressed between the flange 44 of the sleeve 30 and the bottom 346 of the cavity C34. The spring 32 lies against the flange 44 by its most proximal coil 322 and against the bottom 346 by its distal-most coil 324.

The sleeve 30 is also provided with two pairs of external reliefs, namely a first pair of external reliefs 46 located next to a distal end 48 of the sleeve 30 and a second pair of external reliefs 50 located next to a proximal end 52 of the sleeve 30, opposite to the distal end 48. The flange 44 is located close to the second pair of external reliefs 50 and to the proximal end 52.

The body 34 of the exemplary embodiment includes two pair of clips 54A and 54B, respectively a pair of proximal clips 54A and a pair of distal clips 54B, which may respectively cooperate with the reliefs 50 and 46. The proximal clips 54A are disconnected from the reliefs 50 after activation by the plunger rod head 18, in order to release the spring 32 and activate a safety feature by which the sleeve 30 is moved in a proximal direction along the longitudinal axis A2, together with the syringe 4. At the end of this movement the distal clips 54B and the external reliefs 46 are engaged to lock the sleeve 30 to the body 34. This provides an even higher level of safety as, in the locking position, the sleeve 30 cannot be moved in a distal direction along the longitudinal axis A2 and the needle 12 cannot be exposed again.

The body 34 is also provided with two finger flanges 56, integral with the rest of the body 34. These two flanges respectively define two bearing surfaces S56 where the user of the drug delivery device 2 can press the tips of his/her fingers, preferably the index and middle fingers, when pressing on the end surface S18 with his/her thumb.

Advantageously, the body 34 is also provided, next to its distal end 58 with a row of external reliefs 60 which allow . securing the guiding member 24 to the body 34.

A34 denotes a longitudinal axis of the body 34. A36 denotes a longitudinal axis of the plunger rod cover 36. When the plunger rod cover 36 is mounted on the body 34, that is in an assembled state of the safety system 40, the two axes A34 and A36 coincide with the longitudinal axis A40.

The plunger rod cover 36 has a generally tubular shape. Advantageously, a wall 62 of the plunger rod cover 36 does not extend on a full circumference around the longitudinal axis A36 but is provided with a longitudinal slit or opening O36, which extends all the way along the longitudinal dimension of the plunger rod cover 36. Thus, the plunger rod cover 36 has the shape of a portion of a cylinder, open on one angular sector corresponding to the slit O36.

In the exemplary embodiment of the figures, the cross-section of the plunger rod cover wall 62 is not circular, but includes a portion of a circle, which defines an inner diameter D36 of the plunger rod cover 36, and two straight sections located on either side of the slit O36. W36 denotes the width of the opening O36, measured, perpendicularly to the longitudinal axis A36, between the straight sections.

Advantageously, the diameter D18 is smaller, in particular strictly smaller, than the diameter D36, so that the head 18 can slide within an internal volume V36 of the plunger rod cover 36 with a small gap between these elements. In other words, the ratio D18/D36 is smaller, in particular strictly smaller, than 1.

Advantageously, the ratio D18/D36 is close to one, in particular larger than 0.80, preferably larger than 0.90, more preferably equal to about 0.95, which limits the risk of bending of the plunger rod 10 within the volume V36.

The diameter D18 is also preferably smaller, in particular strictly smaller, than the width W36, so that the head 18 can cross the slit O36, when the plunger rod cover 36 is separated from the body 34.

According to a variant of the invention non-represented in the drawings, a proximal end 622 of the wall 62 may be equipped with a closing wall, substantially perpendicular to the longitudinal axis A36 and preventing an axial action on the head 18, along the longitudinal axes A2 and A40.

Close to its distal end 64, the plunger rod cover 36 has an internal skirt 66, which is extends on a limited angular sector around the longitudinal axis A36, where the cross section of the wall 62 is circular. The internal skirt 66 guides a relative rotation between the body 34 and the plunger rod cover 36, around the longitudinal axis A40.

The plunger rod cover 36 also includes an outer peripheral collar 68 which surrounds a distal end 624 of the wall 62 and which is supposed to come into abutment against a proximal edge 70 of the body 34, when the parts 34 and 36 are coupled together.

Two structures 72 extend axially from the collar 68 along the longitudinal axis A36, opposite to the wall 62. Each axially extending structure 72 includes, on its end opposite to the collar 68, a ledge 74 which is perpendicular to, and oriented toward, the longitudinal axis A36. Each ledge 74 defines a proximal surface S74, also perpendicular to the axis A36.

Each axially extending structure 72 comprises a through-hole 76 formed in a peripheral wall 78, which connects its ledge 74 to the collar 68.

In the embodiment presented on the figures 1 to 6, each axially extending structure 72 is formed of a ledge 74 and a wall 78.

In a variant of the invention non-represented in the drawings, each through-hole 76 can be replaced by a blind hole with its opening oriented toward the longitudinal axis A36, thus toward the longitudinal axis A40 when parts 34 and 36 are coupled together.

On the other hand, the body 34 is provided, close to its proximal end defined by the edge 70, with two deformable tabs 80, which protrude from an outer peripheral cylindrical surface S34 of the body 34, which is defined by the main portion 348. Each tab 80 is arc-shaped and is attached by its two ends 82 and 84 to the main portion 348 of the body 34, while it is separated from this main portion, between these two ends 82 and 84, by a gap G80. This gap allows each tab 80 to be flexible and to deform, preferably elastically, under a stress in a radial and inwardly oriented direction, with respect to the longitudinal axis A34. A radial and inwardly oriented direction can also be named a centripetal direction.

In a variant of the invention non-represented in the drawings, each arc-shaped tab 80 is attached to the outer peripheral cylindrical surface S34 at only one of its two ends 82 or 84.

Advantageously, each tab 80 is integral with the main portion 348 of the body 34.

Each tab 80 is defined, along the longitudinal axis A34, between a proximal surface S80 and a distal surface S'80. The proximal surface S80 is preferably parallel to the edge 70 and located, along the longitudinal axis A34, at the same level as the edge 70. The distal surface S'80 is also parallel to the edge 70 but offset to this edge, along the longitudinal axis A34, in the direction of the distal end 58. The distal surface S'80 is oriented toward the finger flanges 56. Both surfaces S80 and S'80 are preferably perpendicular to the longitudinal axis A34.

Advantageously, each tab 80 is provided with a protrusion 86 which extends in a radial outwardly oriented direction with respect to the longitudinal axis A34, thus with respect to the longitudinal axis A40 when the body 34 is connected with the plunger rod cover 36. A radial and outwardly oriented direction can also be named a centrifugal direction.

Advantageously, a protrusion 86 is located, along the circumference of the edge 70, between the two ends 82 and 84 and in the middle of the arc-shaped part of the tab connecting these ends of the corresponding tab 80.

Advantageously, each protrusion 86 is integral with the corresponding tab 80.

Two stops 88, preferably integral with main portion 348 of the body 34, are provided on the outer peripheral surface S34.

In the assembled configuration represented in figure 1, the plunger rod cover 36 protects the plunger rod 10 and the head 18 from an unintended displacement along the longitudinal axis A2, from bending and from any other distortion.

Assembly and disassembly between the body 34 and the plunger rod cover 36 of the safety system 40 occurs in the presented embodiment by relative rotation of these parts 34 and 36, around the longitudinal axis A40.

The plunger rod cover 36 of the presented embodiment is assembled to the body 34 in the following way: The plunger rod cover 36 can be moved along the longitudinal axis A40 in order to bring its collar 68 in contact with the edge 70 of the body 34 and at the same time to insert its internal skirt 66 into the volume V34. This is made with a relative angular orientation of parts 34 and 36 around the longitudinal axis A40, such that the axially extending structures 72 do not contact the tabs 80.

The safety system 40 is then in the configuration of figure 1.

It is then possible to rotate the plunger rod cover 36 in a direction of connection represented by arrow R_{C} in figure 1, which brings each sliding surface S74 in sliding engagement with one surface S'80 of a tab 80, up to the point where the outer wall of each axially extending structure 72 contacts a protrusion 86. During the rotational movement between parts 36 and 34 in the direction of arrow R_{C}, the walls 78 of the axially extending structures 72 act on the protrusions 86 so that these protrusions are pushed radially toward the longitudinal axis A40, that is in a radial and inwardly oriented direction, due to an elastic deformation of the tabs 80, and do not oppose a further rotational movement of the plunger rod cover 36, in the direction of arrow R_{C}, with respect to the body 34.

Due to this further rotational movement, each through hole 76 becomes radially aligned with the respective protrusion 86 and, due to the flexibility of the tabs 80, the protrusions 86 are pushed back in a radial outwardly oriented direction and move into the through holes 76, which forms recesses for accommodating the protrusions. This couples the two parts 34 and 36 in translation along the longitudinal axis A40 in both directions due to the cooperation of parts 68 and 70, on the one hand, and surfaces S74 and S'80, on the other hand.

Thus, each tab 80 equipped with a protrusion 86 forms a first coupling member arranged on the outer peripheral surface S64 of the body 34, whereas each axially extending structure 72 equipped with a through hole 76 forms a corresponding second coupling member arranged on the plunger rod cover 36.

As explained here above, each through hole 76 forms a recess for accommodating, at least partially, one protrusion 86.

The stops 88 form abutment surfaces for delimiting the end position of the disconnection process described here above. In this way the user is prevented from rotating the plunger rod cover 36 too far, which might result in connecting again the plunger rod cover with the body 34 after a rotation by 180°.

In the configuration where the protrusions 86 are respectively engaged in the through holes 76, the first and second coupling members 80 and 72 are in a coupled configuration, where the body 34 and the plunger rod cover 36 are coupled together along the longitudinal axis A40, by the cooperation of the collar 68 with the edge 70 and of the sliding surfaces S74 with the distal surfaces S'80.

When it is necessary to separate the plunger rod cover 36 from the other elements of the safety system 40, in order to access the plunger rod 10 and to use the syringe 4, a disconnection process can be implemented by rotation of the plunger rod cover 36 with respect to the body 34 around the longitudinal axis A40, in a direction opposition to the direction used for coupling these two parts, namely the direction of arrow R_{D} in figure 1.

Alternatively the rotation directions R_{C} and R_{D} may be reversed with respect to their directions shown in figure 1.

This rotation induces that a side wall of the opening 76 presses on one of two chamfered surfaces 862 and 864, which constitute transition zones between each protrusion 86 and the remaining portion of the corresponding tab 80.

This induces that, each tab 80 elastically deforms, so that its protrusion 86 is moved in a radial and inwardly oriented direction, with respect to the longitudinal axis A40, up to a point where each protrusion 86 is outside from the corresponding through hole 76.

Then, the protrusions 86 do not oppose a further rotational movement of the plunger rod cover in the direction of arrow R_{D}, which allows decoupling parts 34 and 36 from each other.

Thus, a relatively small torque is sufficient to push back each protrusion 86 in a radial inwardly oriented direction, with respect to the longitudinal axis A40, and therefore move each protrusion 86 out of the corresponding recess forming through hole 76. This is possible, in particular, because each tab 80 is configured to deform during the disconnection process. Preferably, each tab 80 is flexible and configured to deform elastically during the disconnection process.

The elastic deformation of the tabs 80 of the first embodiment during the disconnection process is possible due to the geometry of the body 34, in particular due to the geometry of the tabs 80 and to the presence of the gaps G80.

The elastic properties of the tabs can be achieved with any material of the body 34 and/or the plunger rod cover 36 used in the manufacturing process, preferably manufacturing by injection molding.

In the embodiment, said material can be composed mainly of a copolymer of methyl methacrylate, acrylonitrile, butadiene and styrene (MABS), a copolymer of methyl methacrylate butadiene styrene (MMBS), polypropylene (PP), copolymer of acrylonitrile-butadiene-styrene (ABS), polyoxymethylene (POM), polyamide (PA), polybuthylene terephtalate (PBT), a polycarbonate (PC) or their mixtures.

Alternatively, other materials can be considered for manufacturing the body 34 and the plunger rod cover 36.

During the disconnection process, the plunger rod cover 36 is guided in rotation around the longitudinal axis A40 by the cooperation of the surfaces S74 and S'80.

The elastic deformation of the tab 80 makes it easier, for the user of the drug delivery device 2 comprising the safety system 40 according to the invention, to remove the plunger rod cover 36, without having to exert an intense torque. This is all the more important that this action can be made by a patient, who is not always in a good physical condition, or by a nurse or another healthcare professional, who needs to repeat this action many times during a working shift. Thus, decreasing the torque needed to decouple the parts 34 and 36 is significant in terms of comfort of use of the safety system 40.

Advantageously, an angular amplitude of the rotational movement of the disconnection process is less than or equal to 100°, preferably between 30° and 50°. In the example of the figures, this angular amplitude is of about 40°, which is enough to make the ledges 74 of the axially extending structures 72 escape from the tabs 80 at the end of the disconnection process.

Once the rotational movement of the disconnection process brings the axially extending structures 72 out of range of the tabs 80, that is when the coupling means 80 and 72 have been decoupled, the plunger rod cover 36 can be disengaged from the body 34 via an axial movement along the longitudinal axis A40, in the proximal direction.

Thus, the disconnection of the plunger rod cover 36 from the other parts of the drug delivery device 2, including the body 34, takes place due to a rotational movement, then a translation movement, with no risk of bumping into the plunger rod 10 or the head 18.

After the disconnection process, the axially extending structures 72 and the tabs 80 are in a decoupled configuration and the plunger rod cover 36 can be moved in several directions, in particular translated along and rotated around the longitudinal axis A34 of the body 34.

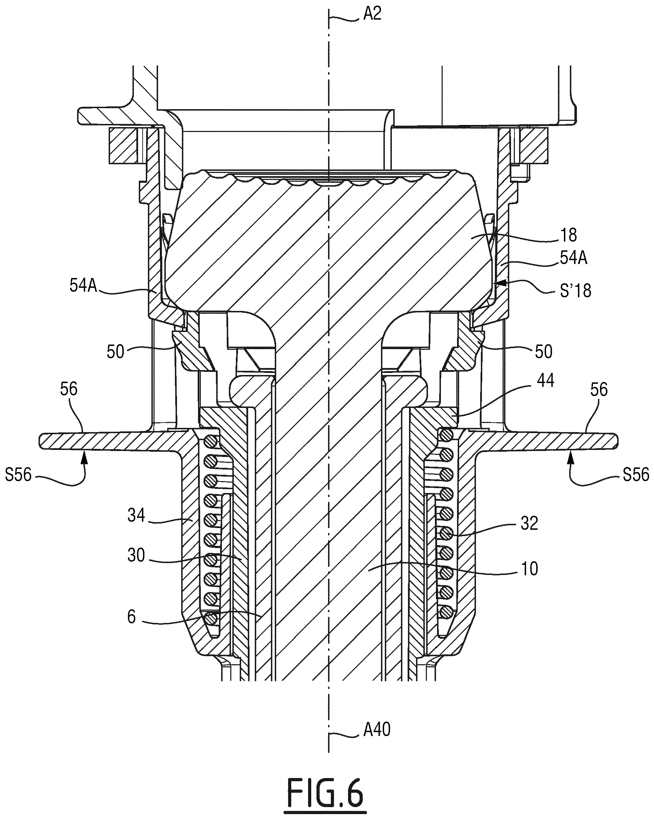

After disconnection of the parts 34 and 36 of the safety system 40, after use of the syringe 4 and once the full dose initially present in the syringe barrel 6 has been expelled out of the internal volume V6, the head 18 activates the connection between the sleeve 30 and the body 34. As shown in figure 6, a peripheral surface S'18 of the head 18 engages the clips 54A. Due to this operation, the sleeve 30 is released and can be moved by the spring 32, in a proximal direction along the longitudinal axis A2. This moves the syringe barrel 6 and the needle 12 in the same direction in the proximal direction and the needle 12 becomes hidden within the body 34. One can make use here of the technical teachings of EP2047879A1.

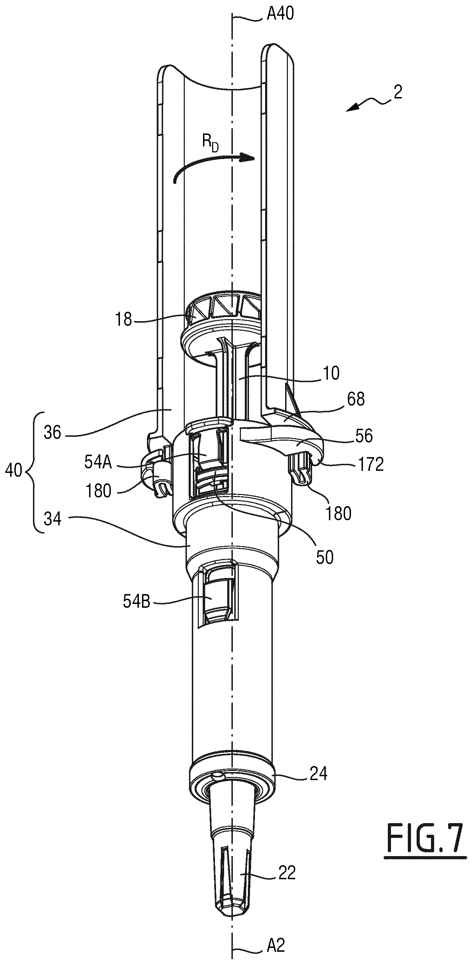

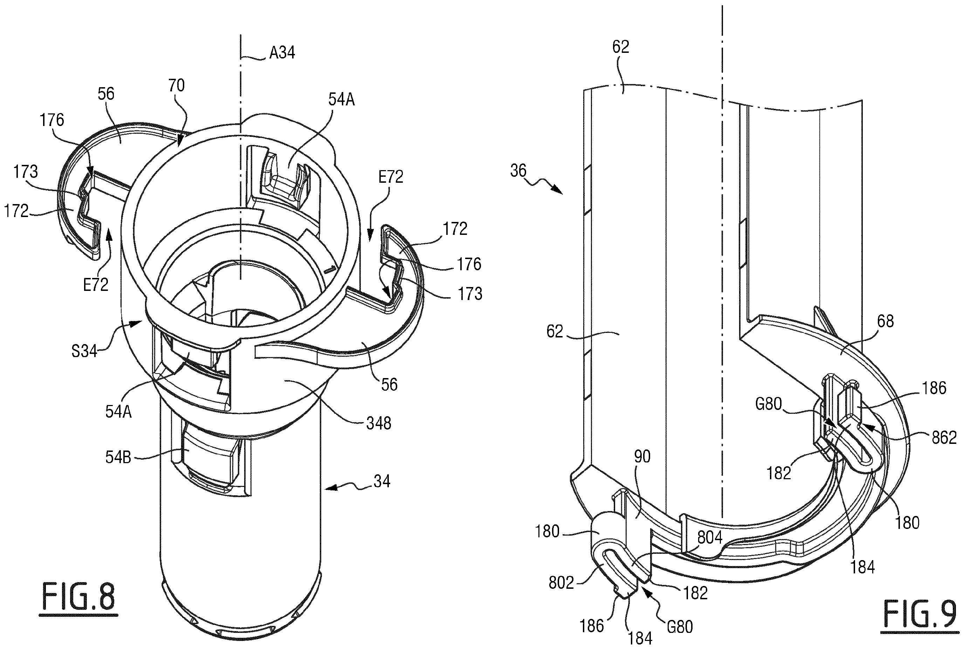

In the second embodiment of the invention represented in figures 7 to 9, the same elements as in the first embodiment have the same references. If a reference is used in the description of the second embodiment without being shown in the figures or is shown in the figures without being mentioned in the description, it corresponds to the same part of as the one having the same reference in the first embodiment.

The following part is focused on the differences between the second embodiment and the first embodiment. The parts identical to, or working in the same way as, the corresponding parts in the first embodiment are not described in detail or omitted in the description.

In the second embodiment, a body 34 and a plunger rod cover 36 together belong to a safety system 40, whose longitudinal axis is noted A40.

In the second embodiment, first coupling members are formed by lateral extensions 172 of the finger flanges 56, which protrude radially from the outer peripheral cylindrical surface S34 of the body 34. In other words, the first coupling member 172 belong to the finger flanges 56 which are integral with the body 34.

Each lateral extension 172 extends opposite to the outer peripheral cylindrical surface S34 of the body 34 and is separated from this surface by an empty space E72, which extends between the lateral extension 172 and the outer peripheral cylindrical surface S34. An edge 173 of each lateral extension 172 oriented toward the outer peripheral cylindrical surface S34 defines a notch 176, which forms a recess and opens in the empty space E72.

On the other hand, the collar 68 of the plunger rod cover 36 is provided with two U shaped tabs 180. Advantageously, each tab 180 is integral with the plunger rod cover 36.

Each tab 180 forms a second coupling member and is connected to a base member 90 at one extremity 182 and its free end 184 is equipped with a protrusion 186 oriented in a radial outwardly oriented direction with respect to the longitudinal axis A36, thus with respect to the longitudinal axis A40 when the body 34 is connected with the plunger rod cover 36.

A chamfered transition surface 862 connects each protrusion 186 to the outer surface of the main portion of the corresponding tab 180.

Advantageously, each protrusion 186 is integral with the associated tab 180 and it extends in a radial outwardly oriented direction, with respect to the longitudinal axis A36.

Thus, the opening of each notch 176, which is oriented toward the longitudinal axis A36, thus toward the longitudinal axis A40 in assembled configuration of the safety system 40, is oriented in a direction opposite to the direction of the protrusions 186.

The elastic properties on the second material could be achieved for any material of the body 34 and/or the plunger rod cover 36 as it is indicated above for the first embodiment.

A gap G80 is formed between the two legs 802 and 804 of each U shaped tab 180.

Each tab 180 of the second embodiment is elastically deformable due to its geometry, in particular due to its U-shape.

In the configuration represented in figure 7, the body 34 and the plunger rod cover 36 of the safety system 40 are coupled together by engagement of the tabs 80 into the empty spaces E72 and engagement of the protrusions 86 into the notches 76, which forms recesses configured to receive these protrusions. In this configuration the plunger rod cover 36 cannot be moved in a distal direction because it is blocked by the collar 68 lying against the edge 70 of the body 34. Similarly the plunger rod cover cannot be moved in a proximal direction, because the curved part of each tab 180 is blocked by the end part of the extension 172. Thus, the body 34 and the plunger rod cover 36 are coupled together along the longitudinal axis A40.

Starting from the position of figure 7, when it is necessary to separate the plunger rod cover 36 from the other parts of the safety system 40 of the invention, the plunger rod cover 36 is rotated, around the longitudinal axis A40, with respect to the body 34, in the direction of arrow R_{D} in figure 7. This induces a disconnection process, where the protrusions 186 are ejected out of the notches 176, which is possible due to the elastic deformation of the tabs 180. More precisely, each tab 180 elastically deforms by bringing its extremities 182 and 184 together, so that its protrusion 186 is moved in a radial inwardly oriented direction, with respect to the longitudinal axis A40 up to a point when each protrusion is outside the corresponding notch 76.

Alternatively the coupling elements 172 and 180 may have the opposite orientation, which induces that the decoupling direction is the opposite to the marked R_{D} in figure 7.

With this respect, the chamfered surfaces 862 help the transition of the protrusions 186 from a configuration where they are engaged within the notches 176 to a configuration where they stay in the empty spaces E72, out of the notches.

Thus, in this second embodiment, the lateral extensions 172 form first coupling members provided on the body 34, whereas the U shaped tabs 180 form second coupling members provided on the plunger rod cover 36 and the notches form recesses for accommodating the protrusions 186 in coupled configuration of the body 34 and the plunger rod cover 36.

For the other aspects, the second embodiment works in the same way as the first embodiment.

After the disconnection, the lateral extensions 72 and the tabs 80 are in a decoupled configuration and the plunger rod cover 36 can be moved in several directions, in particular translated along and rotated around the longitudinal axis A34 of the body 34.

According to a variant of the invention non-represented in the figures, applicable to the two represented embodiments, the extended finger flanges can be provided as a separate part, attachable to the body 34 and preferably attachable on a gripping surface of the main part 348 by any convenient means, e.g. by snapping or gluing. One can make use here of the technical teachings of WO2011/135269A1.

According to another variant of the invention non-represented in the figures, applicable to the two represented embodiments, only one first coupling member can be provided on the body 34 and/or only one second coupling member can be provided on the plunger rod cover 36.

Alternatively, three or more first coupling members can be provided on the body 34 and/or three or more second coupling members can be provided on the plunger rod cover 36.

Mirror solutions, with respect to the ones represented for the two embodiments of the figures can also be considered. Namely, an elastically deformable tab with its two ends attached to a main part can be provided as second coupling member on the plunger rod cover 36 and a corresponding recess can be provided on the body 34. Similarly, a deformable tab, similar to the U shaped tab 80 of the second embodiment, can be provided on the body 34, whereas a corresponding notch is provided on the plunger rod cover 36.

According to another variant of the invention non-represented in the figures, applicable to the two represented embodiments, the protrusions 86 or 186 may extend in a radial inwardly oriented direction, with respect to the longitudinal axis A40 of the safety system 40. The location and geometry of the recesses are adapted accordingly.

In a variant of the invention non-represented in the figures, both coupling members provided on the body and on the plunger rod cover are flexible and elastically deform, at least during the disconnection process.

In a variant of the invention non-represented in the figures, no coupling member elastically deforms during the connection process and the elasticity of the tabs is used only during the disconnection process.

In an alternative variant of the invention at least one coupling member is deformed plastically only during the disconnection process.

The embodiments and variants mentioned in the present specification can be combined in order to generate new embodiments of the invention, in the framework of the attached set of claims.

## Claims

1. A safety system (40) for a syringe (4), the safety system having a longitudinal axis (A40) and comprising
- a body (34) provided with at least one first coupling member (80; 172) arranged on an outer surface (S34) of the body;
- a plunger rod cover (36) provided with at least a second coupling member (72; 180);
wherein
- the body (34) and the plunger rod cover (36) cannot move axially with respect to each other, when the first and second coupling members (80, 72; 172, 180) are in a coupled configuration;
- the body (34) and the plunger rod cover (36) are movable with respect to each other at least in the axial direction, when the first and second coupling members (80, 72; 172, 180) are in a decoupled configuration;
- the first and second coupling members (80, 72; 172, 180) decouple via a relative rotation around the longitudinal axis (A40),
**characterized in that** at least one (80; 180) of the first and second coupling members (80, 72 ; 172, 180) is configured to deform during a disconnection process where the first and second coupling members (80,72; 172, 180) decouple via the relative rotation.

2. The safety system of claim 1, **characterized in that** at least one of the coupling members (80; 180) is configured to deform elastically during a connection process, where the first and second coupling members (80,72; 172, 180) couple via a relative rotation around the longitudinal axis (A40).

3. The safety system of any preceding claim, **characterized in that**
- one (80; 180) of the first and second coupling members (80, 72; 172, 180) includes a radial protrusion (86 ; 186), extending in a radial direction with respect to the longitudinal axis (A40); and
- the other one (72; 172) of the coupling members includes a recess (76 ; 176) configured for accommodating the radial protrusion, when the body (34) and the plunger rod cover (36) are in their coupled configuration.

4. The drug delivery device according to claim 3, **characterized in that** the protrusion (86; 186) is provided on an elastically deformable tab (80 ; 180).

5. The drug delivery device according to claim 4, **characterized in that**
- the two ends (82, 84) of the deformable tab (80) are attached to a main portion of the body (34) or of the plunger rod cover (36);
- the other one of the of the coupling members includes an axially extending structure (72) equipped with a sliding surface (S74) configured to slide, during the disconnection process on a corresponding sliding surface (S'80) of the deformable tab (80);
- the recess (76) is provided in the axially extending structure (72).

6. The safety system according to claim 4, **characterized in that** the protrusion (186) is provided at a free end (184) of a U shaped deformable tab (180) configured to be snapped into an empty space (E72) formed by the other one of the coupling member (172).

7. The safety system according to any one of claims 4 to 6, **characterized in that**, the material of the part (34; 36) which carries the deformable tab (80; 180) is composed mainly of a copolymer of methyl methacrylate acrylonitrile butadiene and styrene (MABS), a copolymer of methyl methacrylate butadiene styrene (MMBS), polypropylene (PP), copolymer of acrylonitrile-butadiene-styrene (ABS), polyoxymethylene (POM), polyamide (PA), polybuthylene terephtalate (PBT), a polycarbonate (PC) or their mixtures.

8. The safety system of any preceding claim, **characterized in that** the plunger rod cover (36) has the shape of a portion of a cylinder, opened on one angular sector (O36).

9. The safety system of any preceding claim, **characterized in that** the plunger rod cover (36) has a non-circular cross section.

10. The safety system according to any preceding claim, **characterized in that** during the disconnection process, an angular amplitude of the rotation of the plunger rod cover (36) with respect to the body (34) is less than or equal to 100°, preferably between 30° and 50°.

11. The safety system according to any preceding claim, further comprising a sleeve (30) and a spring (32), wherein the spring is pre-compressed before activation of the safety system (40).

12. The safety system according to any preceding claim, **characterized in that** the body (34) comprises integral finger flanges (56) or extended finger flanges attached to a gripping surface.

13. The safety system according to claim 12, **characterized in that** the first coupling member (172) belongs to the integral finger flanges (56) or extended finger flanges.

14. A drug delivery device (2) comprising
- a syringe (4) with a syringe barrel (6); a plunger (8) sliding within the barrel and a plunger rod (10) configured to push the plunger during a dose delivery; and
- a safety system (40) according to any preceding claim.

15. The drug delivery device according to 14, **characterized in that** the plunger rod comprises a head (18) and **in that** a maximum transverse dimension (D18) of the head is smaller than an internal diameter (D36) of the plunger rod cover (36).
